(19) Europäisches Patentamt
European Patent Office
Office européen des brevets

(11) Veröffentlichungsnummer: **0 218 150**
**B1**

(12) **EUROPÄISCHE PATENTSCHRIFT**

(45) Veröffentlichungstag der Patentschrift: **27.12.90**

(51) Int. Cl.5: **C 07 H 7/033,** C 11 D 3/22, A 23 L 1/226

(21) Anmeldenummer: **86113217.3**

(22) Anmeldetag: **25.09.86**

# (54) Saccharose-tricarbonsäure und ihre Derivate, Verfahren zur Herstellung von Saccharose-tricarbonsäure und ihren Salzen und ihre Verwendung.

(30) Priorität: **07.10.85 DE 3535720**

(43) Veröffentlichungstag der Anmeldung:
**15.04.87 Patentblatt 87/16**

(45) Bekanntmachung des Hinweises auf die Patenterteilung:
**27.12.90 Patentblatt 90/52**

(84) Benannte Vertragsstaaten:
**AT BE CH DE FR GB IT LI NL SE**

(56) Entgegenhaltungen:
**GB-A- 679 776**

(73) Patentinhaber: **HOECHST AKTIENGESELLSCHAFT**
**Postfach 80 03 20**
**D-6230 Frankfurt am Main 80 (DE)**

(72) Erfinder: **Fritsche-Lang, Wolfram, Dr.**
**Rhönstrasse 7**
**D-6140 Bensheim (DE)**
Erfinder: **Leupold, Ernst Ingo, Dr.**
**Am Zäunefeld 15**
**D-6392 Neu-Anspach (DE)**
Erfinder: **Schlingmann, Merten, Dr.**
**Schneidhainer Strasse 32a**
**D-6240 Königstein/Taunus (DE)**

**EP 0 218 150 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Saccharose-tricarbonsäure und ihre Derivate, wie z.B. Salze, Lactone, Ester, Ester oder Amide, ferner ein Verfahren zu ihrer Herstellung sowie ihre Verwendung.

Es ist bekannt, daß die katalytische Oxidation von Kohlenhydraten, speziell die der darin enthaltenen primären Hydroxymethylgruppen, langsam und in den meisten Fällen nicht einheitlich veläuft. So werden beispielsweise oligomere Kohlenhydrate wie ein verzweigtes Arabinoxylan aus Roggenmehl in 4 Tagen bei 65°C nur zu 4% zu Uronsäuren oxidiert. Zwar wurde auch die Oxidation von Saccharose erwähnt, jedoch wurden dabei keine Angaben über die Isolierung der Reaktionsprodukte gemacht. Es wurde ledliglich festgestellt, daß bei einer angeschlossenen Totalhydrolyse nur gefunden wurde, daß im Hydrolysat geringe Mengen Glucuronsäure enthalten waren.

Es ist auch schon die Oxidation von Saccharose mit einem Platin-Tonerde-Katalysator und Sauerstoff beschrieben worden (DE—PS 886305, Beispiel 3 bzw. die entsprechende US—PS 2845439 und GB—A—679776, jeweils Beispiel 4). Diese führte nach 6-stündiger Behandlung nur zu 60% der theoretisch erwarteten Ausbeute an dem entsprechenden Glucuronsäurederivat. Die Isolierung dieser Verbindung wird hingegen nicht beschrieben.

Gegenstand der Erfindung ist nun die Saccharose-tricarbonsäure (das ist (β-D-Arabino-furanarsäure-2-hexulosyl)-α-D-glucopyranosiduronsäure) der Formel

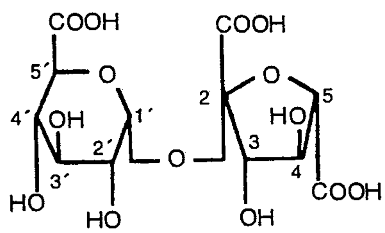

(I)

Ein weiterer Gegenstand der Erfindung ist ein Verfahren zur Herstellung der Saccharose-tricarbonsäure und ihrer Salze, das dadurch gekennzeichnet ist, daß man Saccharose mit Sauerstoff, gegebenenfalls im Gemisch mit inerten Gasen, z.B. in Form von Luft, mit Hilfe eines Katalysators oxidiert, der ein Platinmetall auf Aktivkohle enthält und wesentlich effektiver ist als Platin/Tonerde, wobei der Katalysator 5 bis 10 Gew.-% Metall enthält. Als Katalysatoren eignen sich solche von Platinmetallen, wie Palladium, insbesondere aber Platin selbst, vor allem auf den aktiveren Typen von Aktivkohle. Dabei wird neben einer deutlichen Erhöhung der Oxidationsgeschwindigkeit eine gesteigerte Selektivität der Oxidation zwischen primären und sekundären Hydroxylgruppen der Kohlenhydrate, insbesondere von nichtreduzierenden Kohlenhydraten, erreicht. Bevorzugt führt man die Oxidation in der Weise durch, daß fester Katalysator in einem wäßrigen Reaktionsmedium mit gasförmigen Sauerstoff behandelt wird, d.h. in einer Dreiphasenreaktion. Diese führt man z.B. in einem Blasensäulenreaktor durch, der sowohl diskontinuierlich als auch kontinuierlich betreiben werden kann. Nach bevorzugten Ausführungsformen wird hochkonzentrierter Sauerstoff verwendet und die Reaktionslösung im Kreislauf geführt, wodurch es erleichtert wird, den pH-Wert einzustellen bzw. konstant zu halten. Es liegt im Können des Fachmanns, die Reaktionslösung auf eine gewählte bzw. geeignete Reaktionstemperatur einzustellen und das Konzentrationsverhältnis Substrat/Katalysator bzw. Substrat/Sauerstoff optimal zu bestimmen.

Das erfindungsgemäße Verfahren wirdim allgemeinen bei 30°C bis zum Siedepunkt, vorzugsweise 50 bis 95, insbesondere 60 bis 90°C durchgeführt. Im allgemeinen arbeitet man bei Atmosphärendruck, jedoch ist auch die Anwendung von Überdruck möglich, wodurch das Angebot von Sauerstoff und/oder die Reaktionstemperatur erhöht werden kann. Bei dem erfindungsgemäßen Verfahren ist die Einhaltung bestimmter Saccharose-Konzentrationen besonders vorteilhaft; unter 5 Gew.-% findet leicht ein Überoxidation statt und über 20 Gew.-% lassen sich bei Atmosphärendruck nur verhältnismäßig niedrige Umsätze erzielen. Im allgemeinen arbeitet man bei pH-Werten von 5 bis 9, vorzugsweise 6 bis 8. De Verlauf der Reaktion kann durch Probenentnahmen, z.B. mit gaschromatographischer Analyse de derivatisierten, z.B. silylierten Produkte verfolgt werden.

Die gebildete Saccharose-tricarbonsäure fällt im allgemeinen im Gemisch mit weniger oxidierten Vorstufen an, das sind verschiedene Mono- und/oder Dicarbonsäuren. Man kann die Oxidation schon bei mindestens 20% Tricarbonsäuregehalt abbrechen. Gewöhnlich führt man sie aber so weit, daß mindestens 30 oder 40 und vorzugsweise mehr als 60 oder 70% Saccharose-tricarbonsäure gebildet werden. Diese kann aus dem Reaktionsgemisch in für den Fachmann üblicher Weise konzentriert und isoliert werden.

Das erfindungsgemäße Verfahren kann z.B. in einen doppelmanteligen Reaktor ausgeführt werden, in dem sich die Suspension des Katalysators in dem wäßrien Medium befindet und der unten eine Fritte oder eine entsprechend geeignete andere poröse Membran enthält und von der Unterseite mit einem durch diese Trennmembran feinstverteilten Gasstrom durchströmt wird. Aus wirtschaftlichen und sicherheitstechnischen Gründen wird der Sauerstoff zweckmäßig mti einer solchen Geschwindigkeit durch das Reaktionsmedium geleitet, daß der katalytisch aktivierte Sauerstoff am oberen Ende der Blasensäule

eben verbraucht ist. Zur Verbesserung der Durchmischung und Verlängerung der Einwirkungszeit des Sauerstoffs kann es vorteilhaft sein, das Reaktionsgemisch zu rühren.

Die gebildete Saccharose-tricarbonsäure eignet sich als solche oder in Form ihrer unmittelbar anfallenden Reaktionsprodukte, das sind insbesondere Gemische mit den Vorstufen, auf Grund ihrer chemischen Struktur(elemente) für Anwendungen im Bereich der Komplexbildner, z.B. analog der Glukonsäure und der Glukarsäure in Waschmittelformulierungen, als Lebensmittelzusatzstoffe, z.B. die für Zitronensäure üblichen Anwendungen, als mehrfunktionelle Reaktionspartner (Vernetzung) sowie als Ausgangsmaterial für chemische Umsetzungen (Hydrophilisierungsreagenz).

Durch das erfindungsgemäße Verfahren ist es möglich geworden, die Oxidation von Saccharose dergestalt durchzuführen, daß die bisher nicht bekannte Saccharose-tricarbonsäure entsteht.

## BEISPIELE

1) In einem von außen beheizten, senkrecht angeordneten Glasrohr (Durchmesser: 50 mm, Länge: 80 cm) mit einem Frittenboden und einer wenig darüber installierten Ablaßmöglichkeit für die Reaktionsmischung wird die Lösung von 120 g Saccharose in 1,2 l Wasser sowie 60 g zugesetztem Platin-Katalysator (5% Pt/Aktivkohle) von einem Sauerstoffstrom (ca. 25 Nl/h) von unten nach oben durchströmt. Die durch die Oxidation entstehenden Säuren werden entweder durch Einspeisen von Natronlauge mit der damit verbundenen Möglichkeit der pH-Steuerung oder durch die entsprechenden molaren Mengen an vorgelegtem Natriumhydrogencarbonat ganz oder teilweise in die Natriumsalze überführt. Bei einer Temperatur von 80°C bei der Oxidation und Neutralisation und dabei konstant gehaltenem pH-Wert von 6,5 ergeben sich laut gaschromatographischer Analyse nach Silylierung die folgenden Zusammensetzungen in Abhängigkeit von der Reaktionszeit (s. Tab. 1):

TABELLE 1
Zusammensetzung der Oxidationsprodukte in Abhängigkeit von der Reaktionszeit (in Prozent)

| | 6 h | 12 h | 18 h |
|---|---|---|---|
| Saccharose | verbraucht | — | — |
| Monocarbonsäuren (2 Isomere) | 20,9 | 5,2 | 1,5 |
| Dicarbonsäuren (2 Isomere) | 38,9 | 39,6 | 29,7 |
| Tricarbonsäure I | 5,1 | 22,3 | 35,3 |

Die Reaktionslösung wird vom Katalysator filtriert, über einen Dünnschichtverdampfer eingeengt und gefriergetrocknet. Ausbeute 104 g (86,7 Gew.-%). Der Gesamtsäuregehalt beträgt 7,18 mval/g (theoretischer Wert für Saccharose-tricarbonsäure 7,80 mval/g).

Zur Charakterisierung der Saccharose-tricarbonsäure (STA) wird die Reaktionsmischung mit Hilfe eines handelsüblichen Kationenaustauschers in die freien Säuren übergeführt und nach Filtration gefriergetrocknet. Nach vollständiger Acetylierung mit einem Überschuß von Acetanhydrid und äquimolaren Mengen p-Toluolsulfonsäure (20°C, 20 h) wird der sirupöse Rückstand über Kieselgel chromatographiert (Elutionsmittel: Methylenchlorid/Methanol 10:1 v.v.). Die dabei zuletzt mit polarem Elutionsmittel anfallende pentaacetylierte Saccharose-tricarbonsäure wird mit katalytischen Mengen Natriummethanolat in Methanol in das Trinatriumasalz umgewandelt.

$^1$H—NMR ($D_2O$, 400 MHz): = 5,45 (d, H—1', $J_{1',2'}$ = 3,75 Hz), 4,23—4,12 (m, H—5', H—3, H—4, H—5), 3,82, (strukt, t, H—3', $J_{3',4'}$ = 9,5 Hz, $J_{2',3'}$ = 9,8 Hz), 3,54 (dd, H—2', $J_{1',2'}$ =3,75 Hz, $J_{2',3'}$ = 9,8 Hz), 43,45 (strukt. t, H—4', $J_{3',4'}$ = $J_{4',5'}$ = 9,5 Hz).

GC—MS (Gaschromatographie-Massenspetroskopie) (nach Silylierung, chemische Ionisation mit Isobutan) m/z = 961 (M + 1—Fragment de 8-fach silylierten Verbindung, rel. Intensität ca. 0,1%).

FAB—MS (Fast Atom Bombardment) Natriumsalz eingesetzt, Glycerin als Matrix) m/z = 451 ($MH^+$, rel. Intensität 36%).

2) Wie im Beispiel 1 beschrieben, werden 120 g Saccharose, gelöst in 1,2 l Wasser, bei 60°C und pH 7,5 im Sauerstoffstrom oxidiert. Tab. 2 gibt die GC-analytisch bestimmten Zusammensetzungen wieder.

TABELLE 2
Zusammensetzung der Oxidationsprodukte in Abhängigkeit von der Reaktionszeit (in Prozent)

| | 8 h | 28 h | 52 h | 84 h |
|---|---|---|---|---|
| Saccharose | 1,1 | — | — | — |
| Monocarbonsäuren (2 Isomere) | 28,9 | 10,3 | 5,7 | 4,7 |
| Dicarbonsäuren (2 Isomere) | 35,8 | 36,1 | 27,3 | 23,0 |
| Oxalsäure | 5,9 | 10,8 | 12,6 | 10,9 |
| Tricarbonsäure I | 4,6 | 20,7 | 32,7 | 40,9 |

Nach Filtration des Katalysators und Gefriertrocknung des Filtrates erhält man 93,8 g (78,0 Gew.-%).

**Patentansprüche für die Vertragsstaaten: BE CH DE FR GB IT LI NL SE**

1. Saccharose-tricarbonsäure und ihre Derivate.

2. Verfahren zur Herstellung von Saccharose-tricarbonsäure und ihren Salzen, dadurch gekennzeichnet, daß man Saccharose mit Sauerstoff, gegebenenfalls im Gemisch mit inerten Gasen, mit Hilfe eines Katalysators oxidiert, der ein Platinmetall auf Aktivkohle enthält und effektiver ist als Platin/Tonede, wobei der Katalysator 5 bis 10 Gew.-% Metall enthält.

3. Verfahren nach Anspruch 2, dadurch gekennzeichnet, daß man die Reaktion in der Weise durchführt, daß der feste Katalysator in einem wäßrien, gelöste Saccharose enthaltenden Reaktionsmedium mit gasförmigem, vorzugsweise hochkonzentriertem Sauerstoff behandelt wird.

4. Verfahren nach Anspruch 2 oder 3, dadurch gekennzeichnet, daß die Oxidation bei 30°C bis zum Siedepunkt, vorzugsweise 50 bis 95 und insbesondere 60 bis 90°C durchgeführt wird, vorzugsweise bei Atmosphärendruck.

5. Verfahren nach einem oder mehreren der Ansprüche 2 bis 4, dadurch gekennzeichnet, daß das flüssige Reaktionsmedium 5 bis 20 Gew.-% Saccharose enthält.

6. Verfahren nach einem oder mehreren der Ansprüche 2 bis 5, dadurch gekennzeichnet, daß man die Oxidation bei einem pH-Wert von 5 bis 9, vorzugsweise 6 bis 8 durchführt.

7. Verfahren nach einem oder mehreren der Ansprüche 2 bis 6, dadurch gekennzeichnet, daß der Katalysator ein Platin/Aktivkohle-Katalysator ist.

8. Verwendung von Saccharose-tricarbonsäure und ihren Salzen in Waschmitteln bzw. als Lebensmittelzusatzstoff.

**Patentansprüche für den Vertragsstaat: AT**

1. Verfahren zur Herstellung von Saccharose-tricarbonsäure und ihren Salzen, dadurch gekennzeichnet, daß man Saccharose mit Sauerstoff, gegebenenfalls im Gemisch mit inerten Gasen, mit Hilfe eines Katalysators oxidiert, der ein Platinmetall auf Aktivkohle enthält und effektiver ist als Platin/Tonerde, wobei der Katalysator 5 bis 10 Gew.-% Metall enthält.

2. Verfahren nach Anspruch 1, dadurch gekennzeichnet, daß man die Reaktion in der Weise durchführt, daß der feste Katalysator in einem wäßrien, gelöste Saccharose enthaltenden Reaktionsmedium mit gasförmigem, vorzugsweise hochkonzentriertem Sauerstoff behandelt wird.

3. Verfahren nach Anspruch 1 oder 2, dadurch gekennzeichnet, daß die Oxidation bei 30°C bis zum Siedepunkt, vorzugsweise 50 bis 95 und insbesondere 60 bis 90°C durchgeführt wird, vorzugsweise bei Atmosphärendruck.

4. Verfahren nach einem oder mehreren der Ansprüche 1 bis 3, dadurch gekennzeichnet, daß das flüssige Reaktionsmedium 5 bis 20 Gew.-% Saccharose enthält.

5. Verfahren nach einem oder mehreren der Ansprüche 1 bis 4, dadurch gekennzeichnet, daß man die Oxidation bei einem pH-Wert von 5 bis 9, vorzugsweise 6 bis 8 durchführt.

6. Verfahren nach einem oder mehreren der Ansprüche 1 bis 5, dadurch gekennzeichnet, daß der Katalysator ein Platin/Aktivkohle-Katalysator ist.

7. Verwendung von Saccharose-tricarbonsäure und ihren Salzen in Waschmitteln bzw. als Lebensmittelzusatzstoff.

**Revendications pour les Etats contractants: BE SE**

1. Acide saccharose-tricarboxylique et ses dérivés.

2. Procédé pour préparer l'acide saccharosetricarboxylique et ses sels, procédé caractérisé en ce qu'on oxyde le saccharose au moyen d'oxygène, éventuellement en mélange avec des gaz inertes, à l'aide d'un catalyseur qui contient un métal de la famille du platine sur du charbon actif et qui est plus efficace que le platine sur alumine, ce catalyseur renfermant de 5 à 10% en poids de métal.

3. Procédé selon la revendication 2, caractérisé en ce que la réaction est effectuée de telle manière que le catalyseur solide est traité, dans un milieu réactionnel aqueux contenant du saccharose dissous, par de l'oxygène gazeux, de préférence très concentré.

4. Procédé selon l'une des revendications 2 et 3, caractérisé en ce que l'oxydation est effectuée à une température comprise entre 30°C et le point d'ébullition, de préférence entre 50 et 95°C ou, mieux encore, entre 60 et 90°C, de préférence sous la pression atmosphérique.

5. Procédé selon l'une quelconque des revendications 2 à 4, caractérisé en ce que le milieu réactionnel liquide contient de 5 à 20% en poids de saccharose.

6. Procédé selon l'une quelconque des revendications 2 à 5, caractérisé en ce que l'oxydation est effectuée à un pH de 5 à 9, de préférence de 6 à 8.

7. Procédé selon l'une quelconque des revendications 2 à 6, caractérisé en ce que le catalyseur est un catalyseur constitué de platine sur charbon actif.

8. Application de l'acide saccharose-tricarboxylique et de ses sels dans des détergents ou comme additifs pour produits alimentaires.

**Revendications pour l'Etat contractant: AT**

1. Procédé pour préparer l'acide saccharosetricarboxylique et ses sels, procédé caractérisé en ce qu'on oxyde le saccharose au moyen d'oxygène, éventuellement en mélange avec des gaz inertes, à l'aide d'un catalyseur qui contient un métal de la famille du platine sur du charbon actif et qui est plus efficace que le platine sur alumine, ce catalyseur renfermant de 5 à 10% en poids de métal.

2. Procédé selon la revendication 1, caractérisé en ce qu'on effectée la réaction de telle manière que le catalyseur solide est traité, dans un milieu réactionnel aqueux contenant du saccharose dissous, par de l'oxygène gazeux, de préférence très concentré.

3. Procédé selon l'une des revendications 1 et 2, caractérisé en ce que l'oxydation est effectuée à une température comprise entre 30°C et le point d'ébullition, de préférence entre 50 et 95°C ou, mieux encore, entre 60 et 90°C, de préférence sous la pression atmosphérique.

4. Procédé selon l'une quelconque des revendications 1 à 3, caractérisé en ce que le milieu réactionnel liquide contient de 5 à 20% en poids de saccharose.

5. Procédé selon l'une quelconque des revendications 1 à 4, caractérisé en ce que l'oxydation est effectuée à un pH de 5 à 9, de préférence de 6 à 8.

6. Procédé selon l'une quelconque des revendications 1 à 5, caractérisé en ce que le catalyseur est un catalyseur constitué de platine sur charbon actif.

7. Application de l'acide saccharose-tricarboxylique et de ses sels dans des détergents ou comme additifs pour produits alimentaires.

**Claims for the Contracting States: BE CH DE FR GB IT LI NL SE**

1. Sucrosetricarboxylic acid and derivatives thereof.

2. A process for preparing sucrosetricarboxylic acid or a salt thereof, which comprises oxidizing sucrose with oxygen, if desired in a mixture with inert gases, in the presence of a catalyst which contains a platinum metal on activated carbon and is more effective than platinum/alumina, the catalyst containing 5 to 10% by weight of metal.

3. The process as claimed in claim 2, wherein the reaction is carried out by treating the solid catalyst in an aqueous reaction medium containing dissolved sucrose, with gaseous, preferably highly concentrated, oxygen.

4. The process as claimed in claim 2 or 3, wherein the oxidation is carried out at 30°C to the boil, preferably 50 to 95 and in particular 60 to 90°C, preferably under atmospheric pressure.

5. The process as claimed in one or more of claims 2 to 4, wherein the liquid reaction medium contains 5 to 20% by weight of sucrose.

6. The process as claimed in one or more of claims 2 to 5, wherein the oxidation is carried out at a pH of 5 to 9, preferably 6 to 8.

7. The process as claimed in one or more of claims 2 to 6, wherein the catalyst is a platinum/active carbon catalyst.

8. Use of sucrosetricarboxylic acid or a salt thereof, in detergent compositions and as a food additive.

**Claims for the Contracting State: AT**

1. A process for preparing sucrosetricarboxylic acid or a salt thereof, which comprises oxidizing sucrose with oxygen, if desired in a mixture with inert gases, in the presence of a catalyst which contains a platinum metal on activated carbon and is more effective than platinum/alumina, the catalyst containing 5 to 10% by weight of metal.

2. The process as claimed in claim 1, wherein the reaction is carried out by treating the solid catalyst in an aqueous reaction medium containing dissolved sucrose, with gaseous, preferably highly concentrated, oxygen.

3. The process as claimed in claim 1 or 2, wherein the oxidation is carried out at 30°C to the boil, preferably 50 to 95 and in particular 60 to 90°C, preferably under atmospheric pressure.

4. The process as claimed in one or more of claims 1 to 3, wherein the liquid reaction medium contains 5 to 20% by weight of sucrose.

5. The process as claimed in one or more of claims 1 to 4, wherein the oxidation is carried out at a pH of 5 to 9, preferably 6 to 8.

6. The process as claimed in one or more of claims 1 to 5, wherein the catalyst is a platinum/active carbon catalyst.

7. Use of sucrosetricarboxylic acid or a salt thereof, in detergent compositions and as a food additive.